# EUROPEAN PATENT SPECIFICATION

(11) **EP 3 490 423 B1**
(45) Date of publication and mention of the grant of the patent: **25.11.2020**
(21) Application number: 17761114.2
(22) Date of filing: 25.07.2017
(51) Int. Cl.: A47L 23/12

(54) **APPARATUS FOR SANITISING SOLES OF FOOTWEAR**
VORRICHTUNG ZUM ENTKEIMEN VON SCHUHSOHLEN
APPAREIL DE DÉSINFECTION DE SEMELLES DE CHAUSSURES

(30) Priority: 27.07.2016 IT 201600078971
(43) Date of publication of application: 05.06.2019
(73) Proprietor: Brocchetto, Umberto, 30133 Noale (VE) (IT)
(72) Inventor: Brocchetto, Umberto, 30133 Noale (VE) (IT)
(74) Representative: Locas, Davide
(86) International application number: PCT/IB2017/054492
(87) International publication number: WO 2018/020409

(56) References cited:
- WO-A2-02/38029
- US-A1- 2009 098 031
- US-A1- 2010 104 470
- US-A1- 2010 296 970

## Description

The present invention relates to an apparatus for sanitising soles of footwear of the type comprising a supporting structure and a reservoir for a sanitising fluid.

In the context of the reference technical sector, there are known apparatuses in the form of a footboard for cleaning and sanitising shoes by means of a jet of sanitising fluid sprayed onto the footwear.

An example of such apparatuses is described in US patent application US 2015/096597A1, relating to a device including a plurality of nozzle heads that spray sanitising fluid through a platform onto which the footwear is placed.

The device is designed to operate automatically, utilising a series of sensors to activate the nozzles and to spray the fluid contained in a pressurised canister.

Another alternative is described in patent application US 2012/167325, relating to an apparatus for cleaning footwear, comprising a system of brushes, ultraviolet lights and sprayer nozzles for an antibacterial fluid.

These operations are controlled by a programmable timer and are performed by means of a rechargeable battery or an electrical connection that enables the rotation of the motors and the use of a pump to spray the fluid by means of the nozzles.

A similar solution is also described in international patent application WO 2013/001464.

However, these known apparatuses have the drawback of requiring connection to external power sources because otherwise they have extremely limited autonomy.

Furthermore, these apparatuses cannot easily be adapted to the various environments in which they may be applied. It is, in fact, obvious that the features of an apparatus intended to be placed at the entrance to a public place will preferably differ from those of an apparatus intended for domestic use.

Another solution is described in US patent application US 2010/296970, which relates to a device for sanitising shoes, comprising a container in the form of a tray, in which there is a mat impregnated with sanitising fluid that can be released when said mat is compressed by the action of the feet.

However, the action provided by this device is limited to the sole and furthermore the impregnated mat is particularly subject to wear and tear.

Therefore, the technical problem addressed by the present invention is to provide an apparatus for sanitising the soles of footwear, which can overcome one or more of the drawbacks mentioned above in relation to the prior art.

Furthermore, an object of the present invention is to provide an apparatus for sanitising the soles of footwear, which is sufficiently autonomous without the need for connection to external power sources in order to operate.

Another object of the present invention is to provide an apparatus for sanitising the soles of footwear, wherein the mechanism for delivering the sanitising fluid is simple and reliable.

A further object of the present invention is to provide an apparatus for sanitising the soles of footwear that has modular features so as to be easily adaptable to various application methods.

A further object of the present invention is to provide an apparatus for sanitising the soles of footwear that allows sufficiently well-dispersed delivery of the sanitising fluid.

This problem is solved and these objects are achieved by the apparatus for sanitising the soles of footwear according to claim 1.

Preferred features of the invention are defined in the dependent claims.

The present invention has some considerable advantages. The main advantage consists in the fact that the apparatus according to the present invention does not require any external power sources in order to operate, apart from manual action by the user.

This action is, however, perfectly natural and does not require any physical force by the user.

Furthermore, the sanitising fluid is delivered by means of a simple and, consequently, reliable mechanism.

Additionally, the apparatus according to the present invention can be operatively associated with further analogous apparatuses, offering the benefit of modularity. The features and advantages of the invention will be more clearly apparent from the detailed description of some embodiments thereof, illustrated by way of non-restrictive example, with reference to the attached drawings, in which:
Fig. 1 is a perspective view of an apparatus for sanitising the soles of footwear according to the present invention;
Fig. 2 is a perspective view along section II-II of the apparatus for sanitising the soles of footwear in Fig. 1;
Fig. 3 is a detailed perspective view of the apparatus for sanitising the soles of footwear in Fig. 2;
Fig. 4 is a plan view of the apparatus for sanitising the soles of footwear according to the present invention, from which a bearing surface has been removed;
Fig. 5 is a perspective view along section V-V of the apparatus for sanitising the soles of footwear in Fig. 4; and
Fig. 6 and 7 are two perspective views of a variant embodiment of the apparatus according to the present invention.

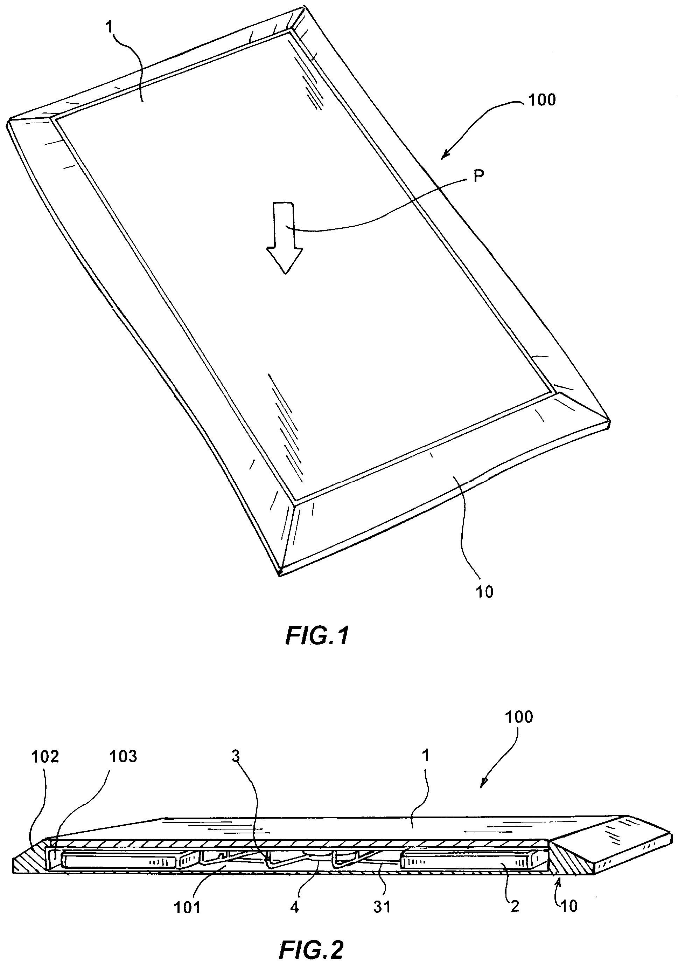

With reference initially to Fig. 1, an apparatus for sanitising the soles of footwear by means of a jet of sanitising fluid is indicated as a whole by reference numeral 100.

It should be noted that, in the context of the present invention, the term "sanitise" does not necessarily involve complete disinfection, but also involves treatment intended to at least partially remove dirt and in particular any bacterial load present. Therefore, the sanitising fluid can also comprise various types of substances for sanitising the sole of the footwear, for example Amuchina®.

In a preferred embodiment, the apparatus 100 is flat and preferably substantially rectangular.

This shape advantageously allows the apparatus 100 to be installed in a similar way to a traditional doormat for cleaning shoes.

As will be more clearly apparent below, the shape and features of the components of the apparatus 100 according to the present invention prove particularly suitable for making said apparatus as flat as possible.

Still in connection to Fig. 1, the apparatus 100 comprises a supporting structure 10, preferably a flat and rectangular support structure, on which a bearing surface 1 is movably supported, which bearing surface is intended to receive, bearing thereon, the sole of the shoe.

As will become clearer below, the bearing surface 1 is movable relative to the supporting structure 10 with respect to a bearing direction P of the foot. In a preferred embodiment, this direction corresponds to the vertical direction, but obviously alternative embodiments can also involve alternative orientations for the bearing direction P, for example tilted.

In one embodiment, the supporting structure 10 comprises a base 101 and a lateral frame 102, which define a housing volume for the components of the apparatus 100, illustrated in detail below.

As can be seen from Fig. 1 and 2, the bearing surface 1 is preferably arranged inside said lateral frame 102. In one embodiment, the bearing surface 1 is flush with one end of the lateral frame 102 and is moved towards the inside of the housing volume as a result of pressure in the bearing direction P.

It is therefore obvious that this pressure can be easily exerted by the action of the foot, using the weight of the user themselves.

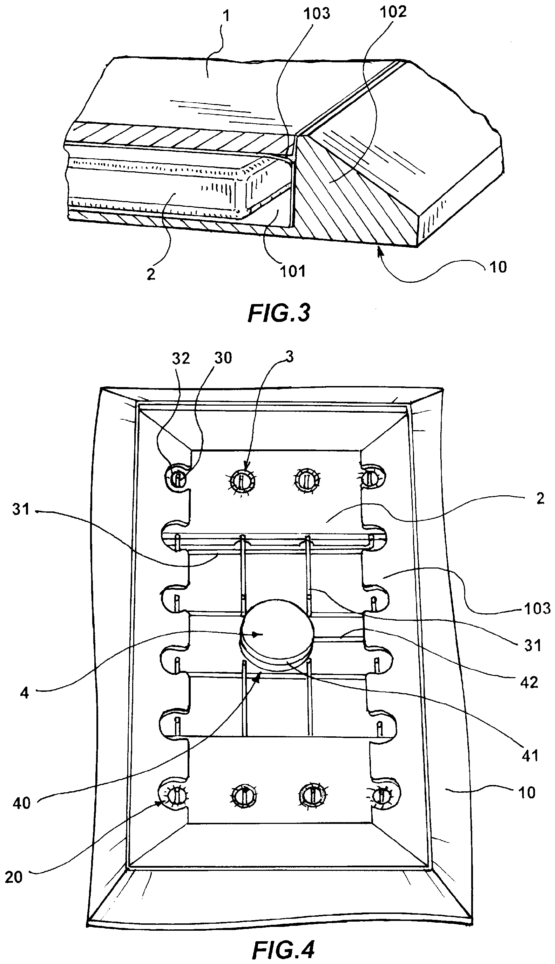

Preferably, the supporting structure 10 comprises an abut member 103, more clearly visible in Fig. 3, for defining an end of travel for the movement of the bearing surface 1 in the bearing direction P.

In one embodiment, the abut member 103 is defined by means of an inner frame that is concentric with the lateral frame 102. Preferably, the abut member 103 has a substantially L-shaped cross section. In this way, a portion of the abut member 103 bears on the lateral frame 102, and a portion perpendicular thereto acts as backing for the bearing surface 1.

Even though alternative solutions can also be provided for the abut member 103, it must be observed that the present embodiment makes it possible to keep as much of the housing volume of the supporting structure 10 available as possible, and at the same time to guarantee sufficient resistance in order to support the weight of a person.

With reference now to Fig. 2 and 4, the apparatus 100 according to the present invention comprises a reservoir 2 for containing sanitising fluid, and a delivery unit 3 for delivering the sanitising fluid towards the bearing surface 1.

Preferably, the reservoir 2 and the delivery unit 3 are housed inside the supporting structure 10, at least in part. Even more preferably, they are both completely housed inside the supporting structure 10.

In a preferred embodiment, the delivery unit 3 comprises spray nozzles 30 facing the bearing surface 1 and spaced apart from one another. The sanitising fluid discharged from the delivery unit can thereby be sprayed towards the bearing surface 1, pass therethrough by methods described in greater detail below, and reach the sole of the footwear worn by the user.

In one embodiment, the spray nozzles 30 are interconnected by means of respective pipes 31. Preferably, the pipes 31 are arranged below the reservoir 2.

In a preferred embodiment, the bearing surface 1 is placed above the reservoir and the delivery unit 3.

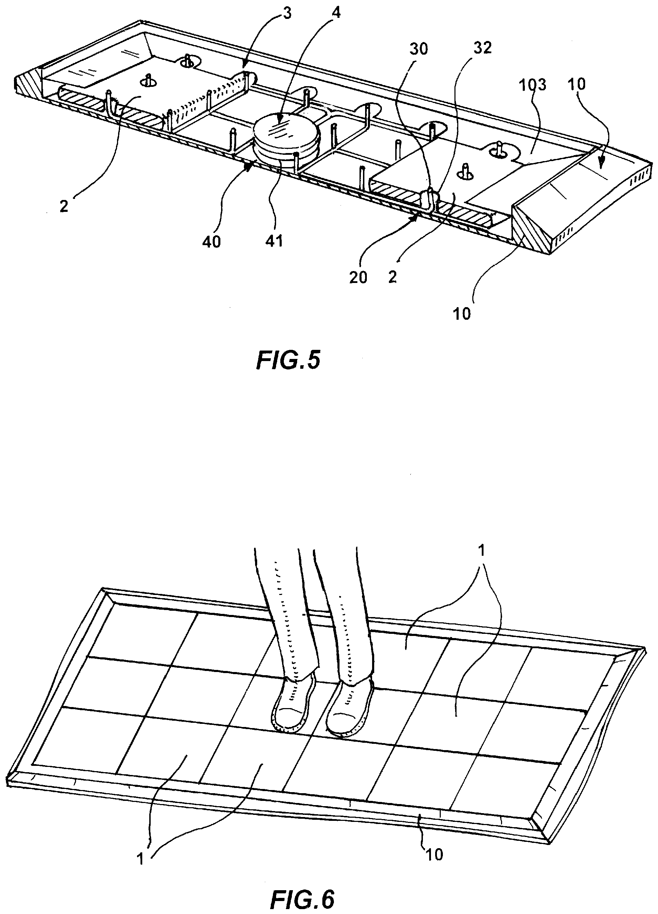

Accordingly, for that purpose, the reservoir 2 comprises a plurality of through-holes 20 for allowing the passage of connecting segments 32 for connecting the pipes 31 to the spray nozzles 30, thus allowing the fluid to be sprayed towards the bearing surface 1, as more clearly visible in Fig. 5.

Preferably, the bearing surface 1 comprises a plurality of through-holes (not visible in the figure), which allow the sanitising fluid to pass through said bearing surface so that said sanitising fluid can reach the sole of the footwear when said footwear is bearing on the surface 1.

The nozzles 30 can thereby be placed below the bearing surface 1, allowing the fluid to pass towards the sole of the footwear in any case.

In a preferred embodiment, the bearing surface 1 comprises a layer made of metal foam having a plurality of interconnected pores suitable for allowing the passage of said sanitising fluid between opposite sides of said bearing surface 1, thus defining the above-mentioned through-holes.

This solution proves particularly advantageous since it combines robustness, simplicity of production and effectiveness in use.

The sanitising fluid is delivered through the nozzles by means of a compressible member 4 that is operatively associable with the bearing surface 1.

In greater detail, the compressible member 4 can be compressed as a result of movement of the bearing surface 1 in the bearing direction P.

To this end, in one embodiment, the compressible member 4 is placed below the bearing surface 1 such that, as a result of the bearing surface 1 being lowered through the action of the foot, said bearing surface comes into contact with the compressible member 4, compressing it.

In a preferred embodiment, the compressible member 4 is connected to the reservoir 2 by means of respective pipes 42, which are partially visible in Fig. 4.

In this way, the variation in volume produced as a result of the compression of the compressible member 4 allows the sanitising fluid present in the reservoir 2 to be forcibly pushed towards the delivery unit 3, consequently delivering it from the respective nozzles 30.

In a preferred embodiment, the compressible member 4 defines a hollow internal portion (not visible in the figures), such that this reduction in volume can take place as a result of the movement of the bearing surface 1 in the bearing direction P. Preferably, the hollow portion is in fluidic communication with the reservoir 2 such that the fluid inside said reservoir can be forcibly pushed by means of the pressure created by the compression of the compressible member 2.

With reference to Fig. 4, the compressible member 4 preferably comprises a casing 41 having elastically deformable walls, which allows the compressible member 4 to be returned to an expanded condition once the action of the foot on the bearing surface 1 is discontinued. In a preferred embodiment, the casing 41 is made of plastics material.

In other words, the compressible member 4 is made like a bellows.

A resilient return member 40 is thereby defined, allowing the compressible member 4 to be restored to the expanded condition after it has been compressed and the fluid has been forcibly pushed towards the nozzles 30.

Obviously, for that purpose, the pipes 42 and 31 will be provided with suitable non-return valves (not illustrated in the figures).

In this way, each time pressure is exerted on the bearing surface 1, a cycle of sanitising fluid discharge takes place, which can thus affect the sole of the footwear bearing on the surface 1.

Once the bearing surface 1 is crossed, the bearing surface 1 can rise again, returning to the initial position and restoring the compressible member to its initial conditions, thus allowing the sanitising cycle to be repeated the next time a user passes over said bearing surface.

It should lastly be noted, that, as illustrated in Fig. 4, in a preferred embodiment, the compressible member 4 is placed in a central region of the supporting structure 10 and is adjacent to the reservoir 2, which is preferably divided into several interconnected parts.

For example, in the present embodiment, in which the supporting structure 10 has a rectangular shape, the reservoir 2 is divided into two parts, between which the compressible member 4 is placed.

The space occupied can thereby be optimised, allowing sufficient autonomy to be provided for an apparatus of limited thickness.

It should be noted that, in one embodiment, the bearing surface has dimensions such as to allow one foot to be supported thereon. For sanitising both the right and left shoes when a person passes over the bearing surface, two paired apparatuses of the type illustrated in Fig. 1 can be used.

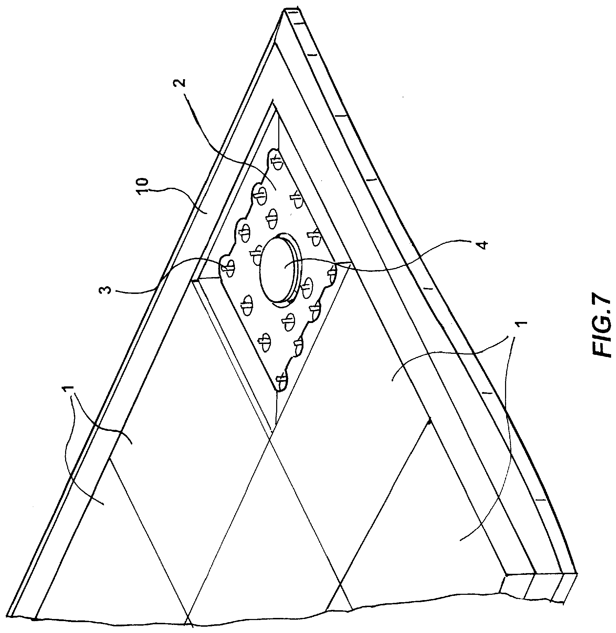

In an alternative embodiment, illustrated in Fig. 6 and 7, the apparatus comprises a plurality of bearing surfaces 1 on a single supporting structure 10. Preferably, the apparatus comprises a plurality of compressible members 4.

Each bearing surface 1 is associated with a respective compressible member 4 and respective nozzles 3 such that only part of the apparatus is involved in the delivery of sanitising fluid.

This embodiment is particularly suitable for use in passageways in public places, for example at the entrance to a hospital, since during transit the user will probably place both his/her feet on a particular bearing surface 1, thus sanitising his/her soles simply by passing over said bearing surface.

The invention therefore solves the proposed problem, simultaneously achieving a plurality of advantages, including the possibility of producing an effective sanitising action without requiring connection to any external power sources or rechargeable sources.

Furthermore, the apparatus can be actuated simply by resting the foot on the apparatus, therefore utilising the user's actual weight. Consequently, this means that said user does not have to exert any force.

Furthermore, in this way the apparatus can also be actuated by simply treading on the bearing surface, as happens naturally when the user passes over it.

Therefore, by placing the apparatus at the entrance to a public place, it is possible to sanitise the soles of the shoes of people going there, with the considerable benefit of cleanliness and hygiene inside that place.

Furthermore, since the apparatus according to the present invention is fully autonomous in its operation, it is possible to use several apparatuses side by side, thus creating a wide treading surface involved in the sanitising action. This therefore also allows the apparatus to be used at the entrance to public places, such as hospitals. In this case, the consumption of sanitising fluid can be optimised by using sufficiently small bearing surfaces for delivery to take place only on the part where footwear is actually placed.

## Claims

1. Apparatus (100) for sanitising soles of footwear, comprising a supporting structure (10), a bearing surface (1) movable in a bearing direction (P) of a user's foot relative to said supporting structure (10), a reservoir (2) suitable for containing sanitising fluid, and a delivery unit (3) for delivering the sanitising fluid towards said bearing surface (1) at a bearing portion for the user's foot, **characterised in that** it further comprises a compressible member (4) operatively associable with said bearing surface (1) in such a way that said compressible member (4) is compressed as a result of a movement of said bearing surface (1) in said bearing direction (P), the compression of said compressible member (4) being suitable for forcing the sanitising fluid contained in said reservoir (2) into said delivery unit (3), and elastic return means (40) suitable for forcing said compressible member (4) into an expanded state.

2. Apparatus (100) for sanitising the soles of footwear according to claim 1, wherein said compressible member (4) defines a hollow internal portion whose volume is reduced as a result of said movement of said bearing surface (1) in said bearing direction (P).

3. Apparatus (100) for sanitising the soles of footwear according to claim 1 or 2, wherein said compressible member (4) comprises a casing (41) with elastically deformable walls that defines said elastic return member (40).

4. Apparatus (100) for sanitising the soles of footwear according to claims 2 and 3, wherein said hollow internal portion is defined by said casing (41).

5. Apparatus (100) for sanitising the soles of footwear according to one of claims 2 to 4, wherein said hollow portion of said deformable member (4) is in fluid communication with said reservoir (2).

6. Apparatus (100) for sanitising the soles of footwear according to anyone of the preceding claims, wherein said delivery unit (3) comprises spray nozzles (30) directed towards said bearing surface (1) and distanced from one another.

7. Apparatus (100) for sanitising the soles of footwear according to claim 6, wherein said spray nozzles (30) are interconnected with one another by means of respective pipes (31), said pipes (31) being arranged below said reservoir (2), said reservoir (2) comprising a plurality of through holes (20) suitable for allowing the passage of connecting segments (32) for connecting said pipes (31) to said spray nozzles (30).

8. Apparatus (100) for sanitising the soles of footwear according to anyone of the preceding claims, wherein said supporting structure (10) comprises a base (101) and a lateral frame (102), said bearing surface (1) being arranged inside said lateral frame (102), said supporting structure (10) further comprising an abut member (103) suitable for defining an end of stroke for said movement of said bearing surface (1) in said bearing direction (P).

9. Apparatus (100) for sanitising the soles of footwear according to anyone of the preceding claims, wherein said abut member (103) is defined by means of an inner frame concentric with said lateral frame (102) and has an essentially L-shaped transverse cross-section.

10. Apparatus (100) for sanitising the soles of footwear according to anyone of the preceding claims, wherein said nozzles are arranged below said bearing surface (1), said bearing surface comprising a layer made of metal foam having a plurality of interconnected pores suitable for allowing the passage of said sanitising fluid between opposing sides of said bearing surface (1).

11. Apparatus (100) for sanitising the soles of footwear according to anyone of the preceding claims, wherein said bearing surface (1) is flush with one end of the lateral frame (102) and is moved towards the inside of a housing volume for the components of the apparatus (100) as a result of pressure in the bearing direction (P).

12. Apparatus (100) for sanitising the soles of footwear according to anyone of the preceding claims, wherein said compressible member (4) is placed below the bearing surface (1) such that, as a result of the bearing surface (1) being lowered through the action of a foot, said bearing surface (1) comes into contact with the compressible member (4), compressing it.

13. Apparatus (100) for sanitising the soles of footwear according to anyone of the preceding claims, wherein said compressible member (4) is in the form of a bellows.

14. Apparatus (100) for sanitising the soles of footwear according to anyone of the preceding claims, wherein said compressible member (4) is placed in a central region of the supporting structure (10) and is adjacent to the reservoir (2).

15. Apparatus (100) for sanitising the soles of footwear according to anyone of the preceding claims, wherein said reservoir (2) is divided into several interconnected parts.

## Patentansprüche

1. Vorrichtung (100) zum Desinfizieren von Schuhsohlen, die eine Stützstruktur (10), eine Auflagefläche (1), die in eine Auflagerichtung (P) eines Fußes eines Benutzers relativ zur Stützstruktur (10) beweglich ist, einen Behälter (2), der zum Aufnehmen einer Desinfektionsflüssigkeit geeignet ist, und eine Zuführeinheit (3) zum Zuführen der Desinfektionsflüssigkeit in Richtung der Auflagefläche (1) an einem Auflagebereich für den Fuß des Benutzers aufweist, **dadurch gekennzeichnet, dass** sie ferner ein komprimierbares Element (4) aufweist, das funktionsmäßig der Auflagefläche (1) zuordenbar ist, so dass das komprimierbare Element (4) infolge einer Bewegung der Auflagefläche (1) in die Auflagerichtung (P) zusammengedrückt wird, wobei die Kompression des komprimierbaren Elements (4) geeignet ist, dass ein Zuführen der Desinfektionsflüssigkeit, die im Behälter (2) enthalten ist, zur Zuführeinheit (3) bewirkt wird, und eine elastische Rückführeinrichtung (40) geeignet ist, um zu bewirken, das komprimierbar Element (4) in einen expandierten Zustand zu bringen.

2. Vorrichtung (100) zum Desinfizieren der Schuhsohlen nach Anspruch 1, wobei das komprimierbare Element (4) einen hohlen inneren Bereich bildet, dessen Volumen infolge der Bewegung der Auflagefläche (1) in die Auflagerichtung (P) verringert wird.

3. Vorrichtung (100) zum Desinfizieren der Schuhsohlen nach Anspruch 1 oder 2, wobei das komprimierbare Element (4) ein Gehäuse (41) mit elastisch verformbaren Wänden aufweist, dass das elastische Rückführelement (40) bildet.

4. Vorrichtung (100) zum Desinfizieren der Schuhsohlen nach Anspruch 2 und 3, wobei der hohle innere Bereich durch das Gehäuse (41) gebildet wird.

5. Vorrichtung (100) zum Desinfizieren der Schuhsohlen nach einem der Ansprüche 2 bis 4, wobei der hohle Bereich des verformbaren Elements (4) in Fluidverbindung mit dem Behälter (2) steht.

6. Vorrichtung (100) zum Desinfizieren der Schuhsohlen nach einem der vorhergehenden Ansprüche, wobei die Zuführeinheit (3) Sprühdüsen (30) aufweist, die auf die Auflagefläche (1) gerichtet und voneinander beabstandet sind.

7. Vorrichtung (100) zum Desinfizieren der Schuhsohlen nach Anspruch 6, wobei die Sprühdüsen (30) mittels jeweiliger Rohre (31) miteinander verbunden sind, wobei die Rohre (31) unter dem Behälter (2) angeordnet sind, wobei der Behälter (2) eine Mehrzahl von Durchgangslöchern (20) aufweist, die geeignet sind, um den Durchgang von Verbindungssegmenten (32) zum Verbinden der Rohre (31) mit den Sprühdüsen (30) zu ermöglichen.

8. Vorrichtung (100) zum Desinfizieren der Schuhsohlen nach einem der vorhergehenden Ansprüche, wobei die Stützstruktur (10) eine Basis (101) und einen seitlichen Rahmen (102) aufweist, wobei die Auflagefläche (1) innerhalb des seitlichen Rahmens (102) angeordnet ist, wobei die Stützstruktur (10) ferner ein Anlageelement (103) umfasst, das zum Bilden eines Bewegungsendes für die Bewegung der Auflagefläche (1) in die Auflagerichtung (P) geeignet ist.

9. Vorrichtung (100) zum Desinfizieren der Schuhsohlen nach einem der vorhergehenden Ansprüche, wobei das Anlageelement (103) mittels eines inneren Rahmens gebildet wird, der konzentrisch zum seitlichen Rahmen (102) ist und einen im wesentlichen L-förmigen Querschnitt aufweist.

10. Vorrichtung (100) zum Desinfizieren der Schuhsohlen nach einem der vorhergehenden Ansprüche, wobei die Düsen unter der Auflagefläche (1) angeordnet sind, wobei die Auflagefläche eine Schicht aus Metallschaum mit einer Mehrzahl von miteinander verbundenen Poren aufweist, die zum Ermöglichen des Durchgangs der Desinfektionsflüssigkeit zwischen gegenüberliegenden Seiten der Auflagefläche (1) geeignet sind.

11. Vorrichtung (100) zum Desinfizieren der Schuhsohlen nach einem der vorhergehenden Ansprüche, wobei die Auflagefläche (1) bündig mit einem Ende des seitlichen Rahmens (102) ist und zur Innenseite eines Gehäusevolumens für die Komponenten der Vorrichtung (100) infolge eines Drucks in Auflagerichtung (P) bewegt wird.

12. Vorrichtung (100) zum Desinfizieren der Schuhsohlen nach einem der vorhergehenden Ansprüche, wobei das komprimierbare Element (4) unter der Auflagefläche (1) angeordnet ist, so dass, infolge der Auflagefläche (1), die durch die Wirkung eines Fußes abgesenkt wird, die Auflagefläche (1) mit dem komprimierbaren Element (4) in Kontakt kommt und es zusammendrückt.

13. Vorrichtung (100) zum Desinfizieren der Schuhsohlen nach einem der vorhergehenden Ansprüche, wobei das komprimierbare Element (4) die Form eines Balgs aufweist.

14. Vorrichtung (100) zum Desinfizieren der Schuhsohlen nach einem der vorhergehenden Ansprüche, wobei das komprimierbare Element (4) in einem zentralen Bereich der Stützstruktur (10) angeordnet ist und neben dem Behälter (2) liegt.

15. Vorrichtung (100) zum Desinfizieren der Schuhsohlen nach einem der vorhergehenden Ansprüche, wobei der Behälter (2) in mehrere, miteinander verbundene Teile unterteilt ist.

## Revendications

1. Appareil (100) de désinfection de semelles de chaussures, comprenant une structure de support (10), une surface d'appui (1) mobile dans une direction d'appui (P) d'un pied d'un utilisateur par rapport à ladite structure de support (10), un réservoir (2) approprié pour contenir un fluide désinfectant, et une unité de distribution (3) pour distribuer le fluide désinfectant vers ladite surface d'appui (1) au niveau d'une portion d'appui pour le pied d'un utilisateur, **caractérisé en ce qu'**il comprend en outre un organe (4) compressible pouvant être associé de manière fonctionnelle à ladite surface d'appui (1) de telle sorte que ledit organe (4) compressible soit compressé à la suite d'un mouvement de ladite surface d'appui (1) dans ladite direction d'appui (P), la compression dudit organe (4) compressible étant appropriée pour forcer le fluide désinfectant contenu dans ledit réservoir (2) dans ladite unité de distribution (3), et un moyen de retour élastique (40) approprié pour forcer ledit organe (4) compressible dans un état déployé.

2. Appareil (100) de désinfection des semelles de chaussures selon la revendication 1, dans lequel ledit organe (4) compressible définit une portion interne creuse dont le volume est réduit à la suite dudit mouvement de ladite surface d'appui (1) dans ladite direction d'appui (P).

3. Appareil (100) de désinfection des semelles de chaussures selon la revendication 1 ou 2, dans lequel ledit organe (4) compressible comprend une enveloppe (41) avec des parois déformables élastiquement qui définit ledit moyen de retour élastique (40).

4. Appareil (100) de désinfection des semelles de chaussures selon les revendications 2 et 3, dans lequel ladite portion interne creuse est définie par ladite enveloppe (41).

5. Appareil (100) de désinfection des semelles de chaussures selon l'une des revendications 2 à 4, dans lequel ladite portion creuse dudit organe (4) déformable est en communication de fluide avec ledit réservoir (2).

6. Appareil (100) de désinfection des semelles de chaussures selon l'une quelconque des revendications précédentes, dans lequel ladite unité de distribution (3) comprend des buses de pulvérisation (30) dirigées vers ladite surface d'appui (1) et à distance les unes des autres.

7. Appareil (100) de désinfection des semelles de chaussures selon la revendication 6, dans lequel lesdites buses de pulvérisation (30) sont interconnectées les unes avec les autres au moyen de tuyaux (31) respectifs, lesdits tuyaux (31) étant agencés en dessous dudit réservoir (2), ledit réservoir (2) comprenant une pluralité de trous débouchants (20) appropriés pour permettre le passage de segments de liaison (32) pour relier lesdits tuyaux (31) auxdites buses de pulvérisation (30).

8. Appareil (100) de désinfection des semelles de chaussures selon l'une quelconque des revendications précédentes, dans lequel ladite structure de support (10) comprend une base (101) et un cadre latéral (102), ladite surface d'appui (1) étant agencée à l'intérieur dudit cadre latéral (102), ladite structure de support (10) comprenant en outre un organe de butée (103) approprié pour définir une fin de course pour ledit mouvement de ladite surface d'appui (1) dans ladite direction d'appui (P).

9. Appareil (100) de désinfection des semelles de chaussures selon l'une quelconque des revendications précédentes, dans lequel ledit organe de butée (103) est défini au moyen d'un cadre intérieur concentrique avec ledit cadre latéral (102) et a une section transversale essentiellement en forme de L.

10. Appareil (100) de désinfection des semelles de chaussures selon l'une quelconque des revendications précédentes, dans lequel lesdites buses sont agencées en dessous de ladite surface d'appui (1), ladite surface d'appui comprenant une couche composée de mousse métallique ayant une pluralité de pores interconnectés appropriés pour permettre le passage dudit fluide désinfectant entre des côtés opposés de ladite surface d'appui (1).

11. Appareil (100) de désinfection des semelles de chaussures selon l'une quelconque des revendications précédentes, dans lequel ladite surface d'appui (1) est dans l'alignement d'une extrémité du cadre latéral (102) et bouge vers l'intérieur d'un volume de boîtier pour les éléments de l'appareil (100) à la suite d'une pression dans la direction d'appui (P).

12. Appareil (100) de désinfection des semelles de chaussures selon l'une quelconque des revendications précédentes, dans lequel ledit organe (4) compressible est placé en dessous de la surface d'appui (1) de telle sorte que, à la suite de l'abaissement de la surface d'appui (1) par l'intermédiaire de l'action d'un pied, ladite surface d'appui (1) entre en contact avec l'organe (4) compressible, le compressant.

13. Appareil (100) de désinfection des semelles de chaussures selon l'une quelconque des revendications précédentes, dans lequel ledit organe (4) compressible est sous la forme d'un soufflet.

14. Appareil (100) de désinfection des semelles de chaussures selon l'une quelconque des revendications précédentes, dans lequel ledit organe (4) compressible est placé dans une région centrale de la structure de support (10) et est adjacent au réservoir (2).

15. Appareil (100) de désinfection des semelles de chaussures selon l'une quelconque des revendications précédentes, dans lequel ledit réservoir (2) est divisé en plusieurs parties interconnectées.
